(19) Europäisches Patentamt
European Patent Office
Office européen des brevets

(11) EP 3 144 304 A1

(12) # EUROPEAN PATENT APPLICATION
published in accordance with Art. 153(4) EPC

(43) Date of publication:
**22.03.2017 Bulletin 2017/12**

(21) Application number: **15792728.6**

(22) Date of filing: **12.05.2015**

(51) Int Cl.:
***C07D 401/04*** *(2006.01)*
***A61K 31/4439*** *(2006.01)*
***A61P 3/10*** *(2006.01)*
***A61P 9/10*** *(2006.01)*
***A61P 9/12*** *(2006.01)*
***A61P 13/12*** *(2006.01)*
***A61P 19/06*** *(2006.01)*
***A61P 43/00*** *(2006.01)*

(86) International application number:
**PCT/JP2015/063632**

(87) International publication number:
**WO 2015/174411 (19.11.2015 Gazette 2015/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **13.05.2014 JP 2014099678**

(71) Applicant: **Teijin Pharma Limited**
**Tokyo 100-0013 (JP)**

(72) Inventors:
- **MIYAMOTO, Hidetoshi**
  **Tokyo 100-0013 (JP)**
- **NOZATO, Hisae**
  **Tokyo 100-0013 (JP)**
- **MARUYAMA, Akinobu**
  **Tokyo 100-0013 (JP)**

(74) Representative: **Carpmaels & Ransford LLP**
**One Southampton Row**
**London WC1B 5HA (GB)**

(54) **NOVEL CRYSTALLINE POLYMORPH OF PYRIDINE DERIVATIVE, AND METHOD FOR PRODUCING SAME**

(57) Disclosed are crystals of 2-(5-chloropyridin-3-yl)-1-(2,5-dichlorobenzyl)-4-methyl-1H-imidazole-5-carboxylic acid useful as therapeutic or prophylactic agents for gout, hyperuricemia, and the like, and a method for producing the crystals.

EP 3 144 304 A1

## Description

[Technical Field]

The present invention relates to crystals of a novel pyridine derivative, which are useful as therapeutic or prophylactic agents for gout, hyperuricemia, and the like, and to a method for producing the crystals.

[Background Art]

Uric acid is the final product resulting from purine degradation in the liver. The primary route through which uric acid in the body is excreted is the kidney, and about two-thirds of it is excreted in the urine, with the remainder excreted in the stool. Blood uric acid levels are maintained in healthy individuals, but, when an excessive production of uric acid or a decreased excretion of uric acid occurs, this causes hyperuricemia.

Hyperuricemia, in which blood uric acid levels become elevated, is a factor that causes gout and urinary calculus, and furthermore it is said to contribute to nephropathy and arteriosclerosis. In addition, there have recently been an increasing number of reports describing that the higher the blood uric acid level, the higher the incidence rates of lifestyle-related diseases such as metabolic syndrome and hypertension, chronic kidney disease, and the like, and hyperuricemia is being recognized to be a risk factor for these diseases. Thus, an improvement in hyperuricemia is expected to lead to improvements in various diseases (NPL 1).

Recently, the gene (SLC22A12) encoding a human renal urate transporter has been identified. The transporter (urate transporter 1, URAT1) encoded by this gene is a twelve-span transmembrane molecule belonging to the OAT family. Its mRNA is specifically expressed in the kidney, and further, its localization on the luminal side of the proximal renal tubule has been observed in human kidney tissue sections. URAT1-mediated uric acid uptake has been shown by experiments using the Xenopus oocyte expression system. Furthermore, it has been reported that probenecid or benzbromarone, which inhibits URAT1, is useful as a therapeutic or prophylactic agent for hyperuricemia, gout, and the like (NPL 2).

[Citation List]

[Non Patent Literature]

[NPL 1] The Guideline Revising Committee of Japanese Society of Gout and Nucleic Acid Metabolism, ed., Guideline for the management of hyperuricemia and gout, second edition, Medical Review (2010).
[NPL 2] Enomoto A. et al., Nature 417, 447-452 (2002).

[Summary of Invention]

[Technical Problems]

It is an object of the present invention to provide crystals of a novel compound, which are useful as therapeutic or prophylactic agents for gout, hyperuricemia, and the like. It is also an object of the present invention to provide a method by which crystals that are chemically stable and suitable as pharmaceutical crude drugs can be produced with high reproducibility.

[Solution to Problem]

As a result of diligent studies with the above objects, the present inventors have found that the compound represented by the following formula (I):

[Chem. 1]

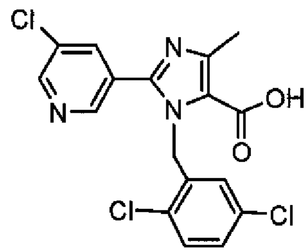

(I)

is capable of crystallization and possesses at least two crystalline polymorphs. They have also found that it is possible to selectively produce the crystalline polymorphs according to the crystallization method.

That is, the present invention provides the following.

[1] A crystal of 2-(5-chloropyridin-3-yl)-1-(2,5-dichlorobenzyl)-4-methyl-1H-imidazole-5-carboxylic acid represented by the following formula (I).

[Chem. 2]

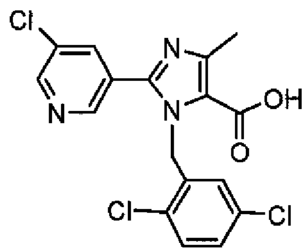

(I)

[2] The crystal according to [1], wherein the crystal has a crystal form A.

[3] The crystal according to [2], wherein the crystal has characteristic peaks at diffraction angles of 2θ = 13.4°, 15.2°, 20.0°, 20.8°, 24.0°, 26.6°, 30.1°, 30.7°, 31.3°, and 33.0° in its X-ray powder diffraction spectrum.

[4] The crystal according to [2], wherein its X-ray powder diffraction spectrum has a pattern shown in Figure 1.

[5] The crystal according to [2], wherein the crystal has characteristic peaks at chemical shifts of 125.8 ppm, 134.8 ppm, 136.7 ppm and 150.1 ppm in its solid-state NMR spectrum ($^{13}C$).

[6] The crystal according to [2], wherein its solid-state NMR spectrum ($^{13}C$) has a pattern shown in Figure 3.

[7] The crystal according to [2], wherein the crystal has characteristic peaks at wave numbers of 1313 $cm^{-1}$, 1324 $cm^{-1}$, and 1391 $cm^{-1}$ in its infrared absorption spectrum (KBr method).

[8] The crystal according to [2], wherein its infrared absorption spectrum (KBr method) has a pattern shown in Figure 5.

[9] The crystal according to any one of [2] to [8], wherein its endothermic peak in thermogravimetry/differential thermal analysis is at 234°C.

[10] The crystal according to [1], wherein the crystal has a crystal form B.

[11] The crystal according to [10], wherein the crystal has characteristic peaks at diffraction angles of 2θ = 10.8°, 12.0°, 13.8°, 16.0°, 19.3°, 21.0°, 22.4°, 23.7°, 26.6°, and 31.2° in its X-ray powder diffraction spectrum.

[12] The crystal according to [10], wherein its X-ray powder diffraction spectrum has a pattern shown in Figure 2.

[13] The crystal according to [10], wherein the crystal has characteristic peaks at chemical shifts of 16.4 ppm (double peak), 120.5 ppm, and 135.6 ppm in its solid-state NMR spectrum ($^{13}C$).

[14] The crystal according to [10], wherein its solid-state NMR spectrum ($^{13}C$) has a pattern shown in Figure 4.

[15] The crystal according to [10], wherein the crystal has characteristic peaks at wave numbers of 1329 $cm^{-1}$, 1382 $cm^{-1}$, and 1401 $cm^{-1}$ in its infrared absorption spectrum (KBr method).

[16] The crystal according to [10], wherein its infrared absorption spectrum (KBr method) has a pattern shown in Figure 6.

[17] The crystal according to any one of [10] to [16], wherein its endothermic peak in thermogravimetry/differential thermal analysis is at 236°C.

[18] A method for producing the crystal of 2-(5-chloropyridin-3-yl)-1-(2,5-dichlorobenzyl)-4-methyl-1H-imidazole-5-carboxylic acid, comprising the steps of:

suspending an alkyl ester of 2-(5-chloropyridin-3-yl)-1-(2,5-dichlorobenzyl)-4-methyl-1H-imidazole-5-carboxylic

acid in a solvent and performing hydrolysis by adding an aqueous solution of a base thereto; and neutralizing the reaction product.

[19] The method for producing the crystal according to [18], further comprising the step of stirring the neutralized product while cooling.
[20] The method for producing the crystal according to [18] or [19], wherein the solvent is a single organic solvent selected from methanol, ethanol, 2-propanol, 1-butanol, and tetrahydrofuran, or a mixture solvent of the organic solvent and water, or a mixture of two or more organic solvents selected from methanol, ethanol, 2-propanol, and 1-butanol, or a mixture solvent of the mixture of the organic solvents and water.
[21] The method for producing the crystal according to [20], wherein the solvent is a single organic solvent selected from methanol, ethanol, 2-propanol, and 1-butanol, or a mixture solvent of the single organic solvent and water.
[22] The method for producing the crystal according to [21], wherein the solvent is ethanol.
[23] The method for producing the crystal according to [18] or [19], wherein the solvent is a mixture solvent of tetrahydrofuran and ethanol.
[24] A pharmaceutical composition comprising the crystal according to any one of [1] to [17] and a pharmaceutically acceptable carrier.
[25] A URAT1 inhibitor comprising the crystal according to any one of [1] to [17] as an active ingredient.
[26] A therapeutic or prophylactic agent for one or more diseases selected from the group consisting of gout, hyperuricemia, hypertension, renal disease, diabetes, arteriosclerosis, and Lesch-Nyhan syndrome, comprising the crystal according to any one of [1] to [17] as an active ingredient.

[Advantageous Effects of Invention]

The present invention provides crystals of a novel pyridine derivative, which are useful as therapeutic or prophylactic agents for gout, hyperuricemia, and the like, and a method for producing the crystals. These crystals can be used as crude drugs for producing medicaments.

[Brief Description of Drawings]

[Figure 1] Figure 1 is a X-ray powder diffraction spectrum of crystal form A.
[Figure 2] Figure 2 is a X-ray powder diffraction spectrum of crystal form B.
[Figure 3] Figure 3 is a solid-state NMR spectrum of crystal form A.
[Figure 4] Figure 4 is a solid-state NMR spectrum of crystal form B.
[Figure 5] Figure 5 is an infrared absorption spectrum of crystal form A.
[Figure 6] Figure 6 is an infrared absorption spectrum of crystal form B.

[Description of Embodiments]

The crystals of the present invention are characterized by X-ray powder diffraction spectra, solid-state NMR spectra, infrared absorption spectra, and/or thermogravimetry/differential thermal analysis (TG/DTA) and the like. The X-ray powder diffraction (XRD) spectra, solid-state NMR spectra, and infrared absorption spectra of these crystals exhibit characteristic patterns, and each crystal has specific diffraction angle 2θ values. In addition, each of these crystals also exhibits its own characteristic thermal behavior in thermogravimetry/differential thermal analysis (TG/DTA).
The crystal form A of the present invention has characteristic peaks at diffraction angles of 2θ = 13.4°, 15.2°, 20.0°, 20.8°, 24.0°, 26.6°, 30.1°, 30.7°, 31.3°, and 33.0° in its X-ray powder diffraction spectrum. In addition, the crystal form A of the present invention has a pattern shown in Figure 1 in its X-ray powder diffraction spectrum.
The crystal form A of the present invention has peaks at chemical shifts of 125.8 ppm, 134.8 ppm, 136.7 ppm and 150.1 in its $^{13}C$ solid-state NMR spectrum. In addition, the crystal form A of the present invention has a pattern shown in Figure 3 in its $^{13}C$ solid-state NMR spectrum.
The crystal form A of the present invention has absorption peaks at wave numbers of 1313 $cm^{-1}$, 1324 $cm^{-1}$, and 1391 $cm^{-1}$ in its infrared absorption spectrum (KBr method). In addition, the crystal form A of the present invention has a pattern shown in Figure 5 in its infrared absorption spectrum (KBr method).
The crystal form A of the present invention has an endothermic peak at 234°C in the thermogravimetry/differential thermal analysis (TG/DTA). The crystal form A is an anhydrous crystal.
The crystal form B of the present invention has characteristic peaks at diffraction angles of 2θ = 10.8°, 12.0°, 13.8°, 16.0°, 19.3°, 21.0°, 22.4°, 23.7°, 26.6°, and 31.2° in its X-ray powder diffraction spectrum. In addition, the crystal

form B of the present invention has a pattern shown in Figure 2 in its X-ray powder diffraction spectrum.

The crystal form B of the present invention has peaks at chemical shifts of 16.4 ppm (double peak), 120.5 ppm, and 135.6 in its $^{13}C$ solid-state NMR spectrum. In addition, the crystal form B of the present invention has a pattern shown in Figure 4 in its $^{13}C$ solid-state NMR spectrum.

The crystal form B of the present invention has absorption peaks at wave numbers of 1329 $cm^{-1}$, 1382 $cm^{-1}$, and 1401 $cm^{-1}$ in its infrared absorption spectrum (KBr method). In addition, the crystal form B of the present invention has a pattern shown in Figure 6 in its infrared absorption spectrum (KBr method).

The crystal B of the present invention has an endothermic peak at 236°C in thermogravimetry/differential thermal analysis (TG/DTA). The crystal form B is an anhydrous crystal.

As used herein, "characteristic peaks" mean peaks which are mainly observed in the X-ray powder diffraction spectrum, $^{13}C$ solid-state NMR spectrum, and infrared absorption spectrum (KBr method) of each crystal polymorph, as well as unique peaks. The crystals of the present invention identified by the peaks of these spectra also include peaks other than those observed as the characteristic peaks described above.

The position and the relative intensity of diffraction angle 2θ in X-ray powder diffraction spectrum may vary somewhat depending on the measurement conditions, and therefore, in the case where 2θ is slightly different, the identity of a crystal form should be recognized by appropriately referring to the pattern of the entire spectrum. Crystals within the range of such errors are also included in the present invention. The errors in 2θ are, for example, in the range of ± 0.5°, and typically in the range of ± 0.2°. In other words, the crystals identified by the above diffraction angles also include those with diffraction angles which coincide in the range of ± 0.5° or ± 0.2°.

Generally, errors can arise also in chemical shifts in $^{13}C$ solid-state NMR spectra. Such errors are in the range, for example, of ± 0.5 ppm, typically ± 0.25 ppm. In other words, the crystals identified by the above chemical shifts also include those with chemical shifts which coincide in the range of ± 0.25 ppm or ± 0.5 ppm.

Generally, errors can arise also in absorption peaks in infrared absorption spectrum (KBr method). Such errors are in the range, for example, of ± 5 $cm^{-1}$, typically ± 2 $cm^{-1}$. In other words, the crystals identified by the above wave numbers also include those with wave numbers which coincide in the range of ± 2 $cm^{-1}$ or ± 5 $cm^{-1}$.

Errors in endothermic peaks in the thermogravimetry/differential thermal analysis (TG/DTA) are, for example, in the range of ± 5°C, typically in the range of ± 2°C. In other words, the crystals identified by the above endothermic peaks also include those with endothermic peaks which coincide in the range of ± 5° or ± 2°.

A crystal of 2-(5-chloropyridin-3-yl)-1-(2,5-dichlorobenzyl)-4-methyl-1H-imidazole-5-carboxylic acid can be produced by a method comprising the steps of: suspending an alkyl ester of 2-(5-chloropyridin-3-yl)-1-(2,5-dichlorobenzyl)-4-methyl-1H-imidazole-5-carboxylic acid in a solvent and performing hydrolysis by adding an aqueous solution of a base thereto ; and neutralizing the reaction product.

In addition, the method for producing a crystal of the present invention can further comprise the step of stirring the neutralized product while cooling.

The crystals of the present invention are obtained by suspending an alkyl ester of 2-(5-chloropyridin-3-yl)-1-(2,5-dichlorobenzyl)-4-methyl-1H-imidazole-5-carboxylic acid in a reaction-inert solvent, including one or more organic solvents selected from methanol, ethanol, 2-propanol, 1-butanol, and tetrahydrofuran, or a mixture solvent of the organic solvent(s) and water, and adding an aqueous solution of a base thereto. The solvent for use in the production of crystal form A includes a single organic solvent selected from methanol, ethanol, 2-propanol, 1-butanol, and tetrahydrofuran, or a mixture solvent of the organic solvent and water, or a mixture of two or more organic solvents selected from methanol, ethanol, 2-propanol, and 1-butanol, or a mixture solvent of the mixture of the organic solvents and water. Preferably, the solvent is a single organic solvent selected from methanol, ethanol, 2-propanol, and 1-butanol, or a mixture solvent of the single organic solvent and water, and more preferably, it is ethanol. When two or more solvents are mixed, the mixing ratio can be appropriately adjusted by a person skilled in the art.

The solvent for use in the production of crystal form B includes a mixture solvent of tetrahydrofuran and ethanol. The mixing ratio of tetrahydrofuran and ethanol can be appropriately adjusted by a person skilled in the art, but 1:1 is preferred.

As the alkyl ester of 2-(5-chloropyridin-3-yl)-1-(2,5-dichlorobenzyl)-4-methyl-1H-imidazole-5-carboxylic acid, C1-C6 alkyl esters are preferred, and ethyl ester is more preferred. As used herein, alkyl esters refer to straight-chain or branched-chain saturated aliphatic hydrocarbon esters. Specific examples of the C1-C6 alkyl esters can include methyl ester, ethyl ester, propyl ester, isopropyl ester, butyl ester, isobutyl ester, tert-butyl ester, pentyl ester, isopentyl ester, hexyl ester, and the like.

The hydrolysis reaction of the alkyl ester of 2-(5-chloropyridin-3-yl)-1-(2,5-dichlorobenzyl)-4-methyl-1H-imidazole-5-carboxylic acid to 2-(5-chloropyridin-3-yl)-1-(2,5-dichlorobenzyl)-4-methyl-1H-imidazole-5-carboxylic acid proceeds by suspending the alkyl ester of 2-(5-chloropyridin-3-yl)-1-(2,5-dichlorobenzyl)-4-methyl-1H-imidazole-5-carboxylic acid in the above solvent, and by adding a base in an amount equivalent to or a slight excess of the alkyl ester of 2-(5-chloropyridin-3-yl)-1-(2,5-dichlorobenzyl)-4-methyl-1H-imidazole-5-carboxylic acid to the suspension. Preferred bases can include sodium hydroxide, potassium hydroxide, or lithium hydroxide. This reaction proceeds at 0°C to 100°C,

but it is preferably carried out at room temperature to 60°C.

After the hydrolysis reaction, neutralization is carried out by reacting the base used with an equivalent or a slight excess of an acid. Preferred acids include hydrochloric acid. The neutralization reaction proceeds at 0°C to 100°C, but it is preferably carried out at 0°C to 60°C.

Then, the neutralized reaction product is stirred while cooling, after which precipitates are collected by filtration and dried to give crystals. Although the stirring conditions and the time that elapses before filtering off the precipitates are not particularly limited, it is preferred that they are set by combining them variously depending on the purpose, since those conditions may affect the yield, chemical purity, particle diameter, particle size distribution of the crystals and the like. For the collection by filtration, a common method, for example, natural filtration, pressure filtration, vacuum filtration, or centrifugation, can be used. For the drying, a common method, for example, natural drying, vacuum drying, heat drying, or vacuum heat drying, can be used.

Synthesis of an alkyl ester of 2-(5-chloropyridin-3-yl)-1-(2,5-dichlorobenzyl)-4-methyl-1H-imidazole-5-carboxylic acid can be performed as shown in Scheme A below, although the synthesis may be performed by any other methods. That is, after the imidazole derivative (V) is brominated to give the compound (IV), N-alkylation is carried out by a reaction using a base and a halide compound, or by a Mitsunobu reaction using an alcohol, to give the compound (III). The alkyl ester (II) of 2-(5-chloropyridin-3-yl)-1-(2,5-dichlorobenzyl)-4-methyl-1H-imidazole-5-carboxylic acid is obtained by a Suzuki coupling reaction of the compound (III) and a boronate derivative. 2-(5-chloropyridin-3-yl)-1-(2,5-dichlorobenzyl)-4-methyl-1H-imidazole-5-carboxylic acid (I) can be obtained by hydrolyzing the ester group of the compound (II).

[Chem. 3]

Scheme A

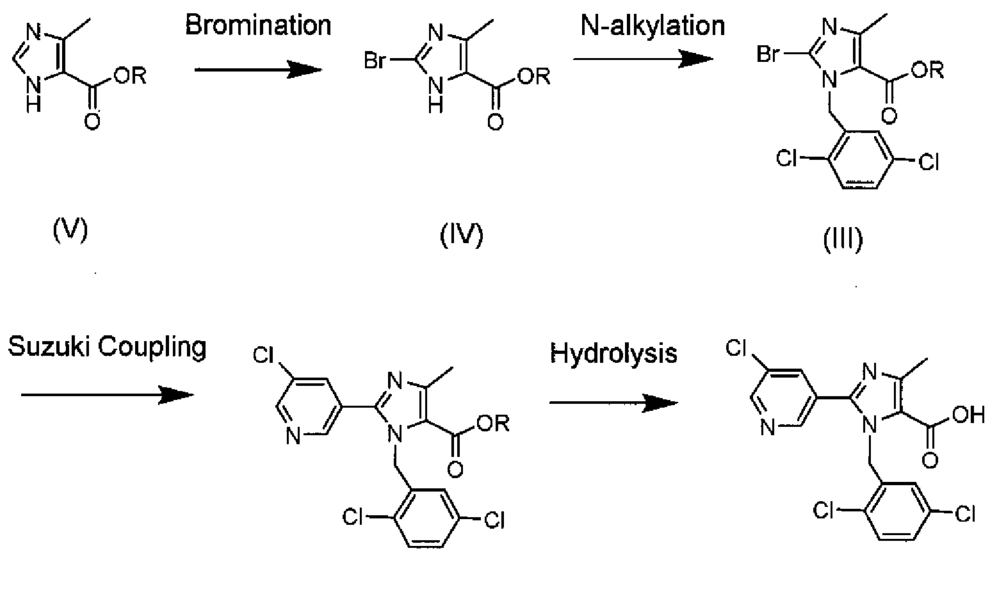


(V) (IV) (III)

(II) (I)

(In the scheme, R is an alkyl group). Suitable reagents for the bromination of the compound (V) to (IV) in Scheme A include bromine, N-bromosuccinimide (NBS), etc. Solvents in this reaction include, but are not particularly limited to, for example, ethers such as tetrahydrofuran (THF), 1,4-dioxane, 1,2-dimethoxyethane, or 1,2-diethoxyethane, halogenated solvents such as dichloromethane or carbon tetrachloride, acetonitrile, mixed solvents thereof, or the like. This reaction proceeds at 0°C to 100°C, but it is preferably carried out at room temperature to 50°C.

The N-alkylation of the compound (IV) to the compound (III) proceeds in a reaction using a base and a halide compound, or by a Mitsunobu reaction using an alcohol. When a base and a halide compound are used, the base includes potassium carbonate, cesium carbonate, triethylamine, diisopropylethylamine, sodium hydride, etc., among which the preferred base is potassium carbonate, cesium carbonate, triethylamine, or diisopropylethylamine. The halide compound includes chloride, bromide, or iodide, among which the preferred halide compound is a chloride or bromide. The temperature for the reaction in the presence of a base and a halide compound is preferably from room temperature to 150°C, and more preferably from 50°C to 120°C. Solvents in this reaction include, but are not particularly limited to, for example, ethers such as tetrahydrofuran (THF), 1,4-dioxane, 1,2-dimethoxyethane, or 1,2-diethoxyethane, amides such as dimethylformamide or N-methylpyrrolidone, dimethyl sulfoxide (DMSO), toluene, xylene, mixed solvents thereof,

or the like. The N-alkylation of the compound (IV) to the compound (III) also proceeds by a Mitsunobu reaction using an alcohol. As for conditions for the Mitsunobu reaction, a phosphine compound, a condensation agent, an alcohol, and the compound (IV) are reacted in an inert solvent to give the compound (III). The phosphine includes tributylphosphine, triphenylphosphine, tricyclohexylphosphine, etc., but preferably triphenylphosphine. A preferred condensation agent is diethyl azodicarboxylate (DEAD) or diisopropyl azodicarboxylate (DIAD). The reaction temperature for this Mitsunobu reaction may be anywhere from 0°C to 100°C, but the preferred reaction temperature is from room temperature to 80°C. The solvent in the Mitsunobu reaction includes, but is not particularly limited to, for example, ethers such as tetrahydrofuran (THF), 1,4-dioxane, 1,2-dimethoxyethane, or 1,2-diethoxyethane, amides such as dimethylformamide or N-methylpyrrolidone, halogenated solvents such as dichloromethane, toluene, xylene, mixed solvents thereof, or the like.

The Suzuki coupling reaction of the compound (III) to the compound (II) proceeds by heating the compound (III), a boronate derivative, a palladium catalyst, and a base in a reaction-inert solvent. Preferably, this reaction is carried out under an inert gas atmosphere. Preferred examples of the boronate derivative include boronic acid and boronic acid pinacol ester. As the palladium catalyst, [1,1'-bis(diphenylphosphino)ferrocene]dichloropalladium (II) ($PdCl_2$(dppf)), tetrakis(triphenylphosphine)palladium ($Pd(PPh_3)_4$), or the like is preferably used. As the base, potassium carbonate, cesium carbonate, or potassium phosphate is mentioned as being preferred. Although the solvent in this reaction is not particularly limited, it is preferable to use, for example, ethers such as tetrahydrofuran (THF), 1,4-dioxane, 1,2-dimethoxyethane, or 1,2-diethoxyethane, amides such as dimethylformamide or N-methylpyrrolidone, alcohols such as ethanol, 2-propanol, or butanol, toluene, xylene, water, or mixed solvents thereof. This reaction proceeds at 50°C to 150°C, but it is preferably carried out at 80°C to 120°C.

The crystals of the present invention can also be obtained by suspending the 2-(5-chloropyridin-3-yl)-1-(2,5-dichlorobenzyl)-4-methyl-1H-imidazole-5-carboxylic acid obtained by the above reaction in a suitable solvent and stirring the suspension. Such a solvent includes methanol, ethanol, 2-propanol, 1-butanol, acetone, methyl ethyl ketone, methyl isobutyl ketone, acetic acid, t-butyl methyl ether, tetrahydrofuran, ethyl acetate, dimethyl sulfoxide, dichloromethane, N,N-dimethylformamide, acetonitrile, toluene, anisole, and water. Although each of the crystal forms of the present invention is distinguishable from the other crystal form by its characteristic X-ray powder diffraction spectrum or thermogravimetry/differential thermal analysis (TG/DTA), this does not refer to a contamination rate of the other crystal form. When a particular crystal form is to be obtained exclusively, at least, such a degree of contamination that cannot be detected by these measurement methods is accepted. In addition, it is also not intended to mean that, when each particular crystal form is used as a crude drug to be formulated as a medicament, inclusion of the other crystal form is not accepted.

Each of the crystal forms of the present invention can be used as an active ingredient of a medicament. Further, they can be used not only as a single crystal form, but also as a mixture of two or more crystal forms.

In the present invention, obtaining 2-(5-chloropyridin-3-yl)-1-(2,5-dichlorobenzyl)-4-methyl-1H-imidazole-5-carboxylic acid as a crystal provides advantages in terms of handling during production, reproducibility, stability, storage stability, and the like, compared to the compound which is not crystalline.

A pharmaceutical composition can be made using a crystal of the present invention and a pharmaceutically acceptable carrier.

A pharmaceutical preparation containing a crystal of the present invention is prepared with additives commonly used to formulate pharmaceutical preparations. Those additives include, in the case of a solid pharmaceutical preparation, an excipient such as lactose, sucrose, glucose, cornstarch, potato starch, crystalline cellulose, light anhydrous silicic acid, synthetic aluminum silicate, magnesium aluminometasilicate, and calcium hydrogenphosphate; a binder such as crystalline cellulose, carboxymethyl cellulose, hydroxypropylcellulose, sodium carboxymethylcellulose, and polyvinylpyrrolidone; a disintegrant such as starch, sodium carboxymethylcellulose, calcium carboxymethylcellulose, croscarmellose sodium, and sodium carboxymethyl starch; a lubricant such as talc and stearates; a coating agent such as hydroxymethylpropylcellulose, hydroxypropylmethylcellulose phthalate, and ethylcellulose; a coloring agent; in the case of a semisolid pharmaceutical preparation, a substrate such as white petrolatum; in the case of liquid pharmaceutical preparation, a solvent such as ethanol; a solubilizing agent such as ethanol; a preservative such as paraoxybenzoate; an isotonization agent such as glucose; a buffering agent such as citrates; an antioxidant such as L-ascorbic acid; a chelating agent such as EDTA; and a suspending agent/an emulsifier such as polysorbate 80; and the like.

The crystals of the present invention can be used in any dosage form such as solid pharmaceutical preparation, semi-solid pharmaceutical preparation, and liquid pharmaceutical preparation, and in any pharmaceutical preparation for application including oral and parenteral preparations (injections, transdermal preparations, ophthalmic solutions, suppositories, nasal preparations, inhalants, and the like).

A pharmaceutical composition containing a crystal of the present invention as an active ingredient can be used as a URAT1 inhibitor, or a therapeutic or prophylactic agent for URAT1-associated diseases, such as gout, hyperuricemia, hypertension, renal disease (such as interstitial nephritis), diabetes, arteriosclerosis, or Lesch-Nyhan syndrome. Here, "preventing" refers to obviating contraction or development of a disease in an individual who has not yet contracted or developed it, and "treating" refers to curing, suppressing, or ameliorating a disease or symptom in an individual who has

already contracted or developed it.

[Examples]

[Measurement Method]

The X-ray powder diffraction of the crystals of the present invention was measured under the following conditions. Apparatus: D8 DISCOVER With GADDS produced by Bruker AXS CS source: Cu-Kα, Wave length: 1.541838 ($10^{-10}$ m), 40 kV-40 mA,
Incident-side flat-plate graphite monochromator, Collimator ϕ 300 μm, Two-dimensional PSPC detector, Scanning 3 to 40°

[Measurement Method]

The solid-state NMR spectra of the crystals of the present invention were measured under the following conditions.

Apparatus: DSX300WB produced by Bruker
Measurement temperature: room temperature Measured nucleus: 13C
Pulse repetition time: 5 seconds
Pulse mode: CP/MAS measurement

[Measurement Method]

The infrared absorption spectra of the crystals of the present invention were measured according to the potassium bromide disk method, which is an infrared spectrum measurement method described in general test methods of the Japanese Pharmacopoeia, under the following conditions.

Apparatus: AVATAR320 produced by Thermo Fisher Scientific.
Measurement range: 4000 to 400 $cm^{-1}$
Resolution: 4 $cm^{-1}$
Integration: 64 times

The thermogravimetry/differential thermal analysis (TG/DTA) of the crystals of the present invention was measured under the following conditions.

Apparatus: TG8120 produced by Rigaku
Heating rate: 10°C/minute, Atmosphere: nitrogen, Sample pan: aluminum, Reference: alumina, Sampling: 1.0 second, Measured temperature range: 20 to 300°C

[Example 1]

Production of crystal form A of 2-(5-chloropyridin-3-yl)-1-(2,5-dichlorobenzyl)-4-methyl-1H-imidazole-5-carboxylic acid

Ethyl 2-(5-chloropyridin-3-yl)-1-(2,5-dichlorobenzyl)-4-methyl-1H-imidazole-5-carboxylate (5.0 g, 11.7 mmol) was suspended in ethanol (32.5 mL), and 2 M aqueous sodium hydroxide solution (11.7 mL, 23.5 mmol) was added thereto. Then, the suspension was stirred at 60°C for one hour and dissolution was confirmed. After the reaction, the reaction product was passed through a filter and washed with water (5.0 mL), followed by neutralization by adding 2 M hydrochloric acid (11.7 mL, 4.4 mmol) over 1 hour or more at 60°C. After crystals were precipitated, stirring was carried out at 60°C for 30 minutes, at room temperature for 30 minutes, and at 0°C for 30 minutes, after which the crystals were collected by filtration. The resultant crystals were washed with a mixture solvent of water (90 mL) and ethanol (10 mL) and vacuum dried overnight at 50°C to give crystals of 2-(5-chloropyridin-3-yl)-1-(2,5-dichlorobenzyl)-4-methyl-1H-imidazole-5-carboxylic acid. The XRD of the resultant crystals is shown in Figure 1. Peaks were observed at diffraction angles of 2θ = 13.4°, 15.2°, 20.0°, 20.8°, 24.0°, 26.6°, 30.1°, 30.7°, 31.3°, and 33.0°. The solid-state NMR spectrum of the resultant crystals is shown in Figure 3 and the infrared absorption spectrum in Figure 5. In addition, the endothermic peak in thermogravimetry/differential thermal analysis (TG/DTA) was observed at 234°C.
$^1$H-NMR (DMSO-d6) δ: 13.13 (1H, s), 8.70 (1H,brs), 8.55(1H,brs), 8.02(1H,brs), 7.50(1H,d,J=8.0Hz), 7.38(1H,dd,J=8.4,2.4Hz) 6.60(1H,brs) 5.60(2H,s), 2.49(3H,s).

[Example 2]

Production of crystal form B of 2-(5-chloropyridin-3-yl)-1-(2,5-dichlorobenzyl)-4-methyl-1H-imidazole-5-carboxylic acid

Ethyl 2-(5-chloropyridin-3-yl)-1-(2,5-dichlorobenzyl)-4-methyl-1H-imidazole-5-carboxylate (1.0 g, 2.35 mmol) was suspended in a mixture solvent of tetrahydrofuran (6.5 mL) and ethanol (6.5 mL), and 2 M aqueous sodium hydroxide solution (2.35 mL,4.7 mmol) was added thereto. Then, the suspension was stirred at 50°C for one hour and dissolution was confirmed. After the reaction, the reaction product was passed through a filter and washed with water (10 mL), followed by cooling to room temperature and neutralization by adding 2 M hydrochloric acid (2.35 mL, 4.7 mmol). After crystals were precipitated, the mixture was cooled to 0°C and stirred for 30 minutes, after which the crystals were collected by filtration. The resultant crystals were washed with a mixture solvent of water (4.5 mL) and ethanol (0.5 mL) and vacuum dried overnight at 45°C to give crystals of 2-(5-chloropyridin-3-yl)-1-(2,5-dichlorobenzyl)-4-methyl-1H-imidazole-5-carboxylic acid (917 mg). The XRD of the resultant crystals is shown in Figure 2. Peaks were observed at diffraction angles of 2θ = 10.8°, 12.0°, 13.8°, 16.0°, 19.3°, 21.0°, 22.4°, 23.7°, 26.6°, and 31.2°. The solid-state NMR spectrum of the resultant crystals is shown in Figure 4 and the infrared absorption spectrum in Figure 6. In addition, the endothermic peak in thermogravimetry/differential thermal analysis (TG/DTA) was observed at 236°C.
$^1$H-NMR(DMSO-d6)δ: 13.13(1H,s), 8.70(1H,brs), 8.55(1H,brs), 8.02(1H,brs), 7.50(1H,d,J=8.0Hz), 7.38(1H,dd,J=8.4,2.4Hz), 6.60(1H,brs), 5.61(2H,s), 2.49(3H,s).

[Reference Example 1]

Production of ethyl 2-(5-chloropyridin-3-yl)-1-(2,5-dichlorobenzyl)-4-methyl-1H-imidazole-5-carboxylate

Ethyl 2-(5-chloropyridin-3-yl)-1-(2,5-dichlorobenzyl)-4-methyl-1H-imidazole-5-carboxylate used in Examples 1 and 2 was produced according to the following method.

[Chem. 4]

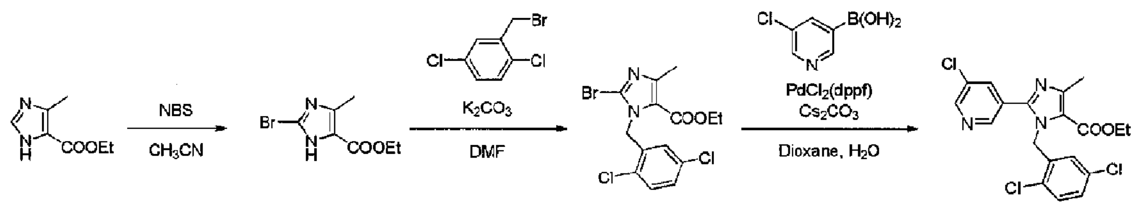


(1) Ethyl 4-methyl-1H-imidazole-5-carboxylate (7.5 g, 48.7 mmol) was dissolved in acetonitrile (120 mL), N-bromosuccinimide (10.4 g, 58.4 mmol) was added thereto, and the mixture was stirred at room temperature for 3 hours. After the reaction, saturated aqueous sodium hydrogen carbonate was added and the mixture was extracted twice with ethyl acetate. After washing the organic layer with saturated aqueous sodium chloride, it was dried over anhydrous sodium sulfate. After concentrating the organic layer, the residue was purified by column chromatography to obtain ethyl 2-bromo-4-methyl-1H-imidazole-5-carboxylate (3.6 g):

$^1$H-NMR($CDCl_3$)δ: 4.35(2H,q,J=7.1Hz), 2.51(3H,s), 1.37(3H,t,J=7.1Hz).

(2) Ethyl 2-bromo-4-methyl-1H-imidazole-5-carboxylate (2.75 g, 11.81 mmol) described in (1) was dissolved in DMF (20 mL), potassium carbonate (3.26 g, 23.62 mmol) and 2,5-dichlorobenzyl bromide (3.4 g, 14.17 mmol) were added thereto, and the mixture was stirred at 90°C for 3 hours. After the reaction, water was added and the mixture was extracted twice with ethyl acetate. The organic layer was washed with saturated aqueous sodium chloride and subsequently dried over sodium sulfate. After concentrating the organic layer, the residue was purified by column chromatography to obtain ethyl 2-bromo-1-(2,5-dichlorobenzyl)-4-methyl-1H-imidazole-5-carboxylate (1.72 g):

$^1$H-NMR($CDCl_3$)δ: 7.34(1H,d,J=8.8Hz), 7.20(1H,dd,J=8.8,2.4Hz), 6.40(1H,d,J=2.4Hz), 5.60(2H, s), 4.25(2H,q,J=7.2Hz), 2.56(3H,s), 1.27(3H,t,J=7.1Hz).

(3) Ethyl 2-bromo-1-(2,5-dichlorobenzyl)-4-methyl-1H-imidazole-5-carboxylate (100 mg, 0.26 mmol), 5-chloropyridin-3-ylboronic acid (30 mg, 0.51 mmol), $PdCl_2$ (dppf) (28 mg, 0.038 mmol), and cesium carbonate (166 mg, 0.51

mmol) were dissolved in a mixed solvent of 1,4-dioxane (0.71 mL) and water (0.14 mL), and the resultant solution was stirred at 95°C over night under a nitrogen atmosphere. After the reaction, water was added and the mixture was extracted twice with ethyl acetate. The organic layer was washed with saturated aqueous sodium chloride and subsequently dried over sodium sulfate. After concentrating the organic layer, the residue was purified by column chromatography to obtain ethyl 2-(5-chloropyridin-3-yl)-1-(2,5-dichlorobenzyl)-4-methyl-1H-imidazole-5-carboxylate (35 mg):

$^1$H-NMR($CDCl_3$)$\delta$: 8.62(1H,d,J=2.0Hz), 8.46(1H,d,J=2.0), 7.89(1H,t,J=2.4Hz), 7.37(1H,d,J=8.4Hz), 7.25(1H,dd,J=9.2,2.6Hz), 6.65(1H,d,J=2.0), 5.55(2H,s), 4.27(2H,q,J=6.8Hz), 2.64 (3H, s), 1.28 (3H, t, J=6.8Hz).

[Reference Example 2]

Production of 2-(5-chloropyridin-3-yl)-1-(2,5-dichlorobenzyl)-4-methyl-1H-imidazole-5-carboxylic acid

Ethyl 2-(5-chloropyridin-3-yl)-1-(2,5-dichlorobenzyl)-4-methyl-1H-imidazole-5-carboxylate (35 mg, 0.0824 mmol) was dissolved in a mixture solvent of tetrahydrofuran (1 mL) and ethanol (0.5 mL), and 2 M aqueous sodium hydroxide solution (0.2 mL, 0.4 mmol) was added thereto. Then, the resultant solution was stirred at 50°C for three hours. After the reaction, 2 M hydrochloric acid (0.2 mL, 0.4 mmol) was added for neutralization, and the neutralized product was concentrated. The residue was purified by conventional methods to give 2-(5-chloropyridin-3-yl)-1-(2,5-dichlorobenzyl)-4-methyl-1H-imidazole-5-carboxylic acid (22 mg).
$^1$H-NMR(DMSO-d6)$\delta$: 8.71(1H,d,J=2.4Hz), 8.56(1H,d,J=2.0Hz), 8.02(1H,t,J=2.0Hz), 7.50(1H,d,J=8.3Hz), 7.38(1H,dd,J=8.8,2.4Hz), 6.62(1H,d,J=2.4Hz), 5.61(2H, s), 2.50 (3H, s).

[Reference Example 3]

Test for inhibition of uric acid transport using human URAT1-expressing cells

2-(5-chloropyridin-3-yl)-1-(2,5-dichlorobenzyl)-4-methyl-1H-imidazole-5-carboxylic acid was dissolved in DMSO (produced by Sigma) to a concentration of 20 mM and the concentration was subsequently adjusted to a value desired for use. Full-length cDNA of human URAT1 (hURAT1) (produced by OriGene Technologies, Inc., NCBI Reference Sequence: NM 144585) was subcloned into an expression vector, pCMV6-Kan/Neo (produced by OriGene Technologies, Inc.), and hURAT1 gene was transfected into human embryonic kidney-derived cells (HEK 293 cells) by liposome method using Lipofectamine 2000 (produced by Invitrogen Corporation), whereupon HEK 293 cells expressing human URAT1 gene were selected on the basis of Geneticin resistance. By a method similar to the following method, functional expression of human URAT1 gene was confirmed by using transport of $^{14}$C -labeled uric acid into the cells as an index.

The HEK 293 cells expressing human URAT1 were seeded in a 24-well cell culture dish to a density of $3\times10^5$ cells/mL/well and were cultured in Dulbecco's modified Eagle's medium (D-MEM medium) containing 10% fetal bovine serum at 37°C for 2 days. Thereafter, the following test for inhibition of uric acid transport was performed.

After the medium was removed by aspiration from each well, the medium was replaced with a solution obtained by substituting NaCl in Hank's Balanced Salt Solution (HBSS) with Na gluconate (hereinafter, HBSS/Na-gluconate) and the cells were preincubated at 37°C for about 10 minutes. HBSS/Na-gluconate was removed by aspiration and a $^{14}$C-uric acid solution that was warmed at 37°C in advance containing various concentrations of the compound and a radioactive ligand ($^{14}$C-labeled uric acid; final concentration 25 $\mu$M) was added and an uptake reaction was carried out by incubating at 37°C for 5 min. After the reaction, $^{14}$C-labeled uric acid solution was removed by aspiration and the cells were washed three times with ice-cold HBSS. The HEK 293 cells expressing human URAT1 were lysed in 0.2 mol/L aqueous NaOH (hereafter, the cell sample) and sampled from the well. The cell sample and a liquid scintillation liquid, ULTIMA GOLD (produced by PerkinElmer, Inc.) were mixed and the radioactivity was measured by a liquid scintillation counter (Beckman Coulter, Inc.).

The uric acid transport rate of the Example compound at each concentration (% of control uptake) was calculated relative to the radioactivity (radioactivity in human URAT1 expressing HEK 293 cells without addition of the compound (DMSO addition)) showing URAT1-specific uric acid transport as 100%, and the concentration ($IC_{50}$) of the compound at which the uric acid transport rate is inhibited by 50% was determined. $IC_{50}$ was found to be 0.2 $\mu$M or less.

[Reference Example 4]

Drug efficacy evaluation test for Cebus apella

2-(5-chloropyridin-3-yl)-1-(2,5-dichlorobenzyl)-4-methyl-1H-imidazole-5-carboxylic acid (3 mg/kg to 30 mg/kg)

dispersed in a 0.5% methylcellulose solution was administered to Cebus apella from the nasal cavity to the stomach using a disposable catheter and a syringe barrel. Blood samples were taken before administration and 30 minutes, 1 hour, 2 hours, 4 hours, 8 hours, 12 hours, and 24 hours after administration; and urine samples were collected for the time intervals of immediately after to 4 hours after administration, from 4 hours to 8 hours after administration, from 8 hours to 16 hours after administration, and from 16 hours to 24 hours after administration. Concentrations of uric acid and creatinine in the blood and urine samples collected were measured by an automatic analyzer (JEOL Ltd.). Uric acid and creatine were measured using L-type Wako UA·F (Wako Pure Chemicals Industries, Ltd.) and L-type Creatine F (Wako Pure Chemicals Industries, Ltd.), respectively. Uric acid clearance was calculated from the uric acid concentrations in blood and urine and, similarly, creatinine clearance was calculated from the creatinine concentrations. From these values, the uric acid excretion rate was determined according to the following equation:

$$\text{Uric acid excretion rate (\%)} = (\text{uric acid clearance/creatinine clearance}) \times 100$$

In the present test, excellent activity to promote uric acid excretion was confirmed for 2-(5-chloropyridin-3-yl)-1-(2,5-dichlorobenzyl)-4-methyl-1H-imidazole-5-carboxylic acid.

[Industrial Applicability]

The crystals of 2-(5-chloropyridin-3-yl)-1-(2,5-dichlorobenzyl)-4-methyl-1H-imidazole-5-carboxylic acid of the present invention are used as medicaments. Furthermore, these crystals can be used as a crude drug for producing medicaments.

## Claims

**1.** A crystal of 2-(5-chloropyridin-3-yl)-1-(2,5-dichlorobenzyl)-4-methyl-1H-imidazole-5-carboxylic acid.

**2.** The crystal according to claim 1, wherein the crystal has a crystal form A.

**3.** The crystal according to claim 2, wherein the crystal has characteristic peaks at diffraction angles of 2θ = 13.4°, 15.2°, 20.0°, 20.8°, 24.0°, 26.6°, 30.1°, 30.7°, 31.3°, and 33.0° in its X-ray powder diffraction spectrum.

**4.** The crystal according to claim 2, wherein its X-ray powder diffraction spectrum has a pattern shown in Figure 1.

**5.** The crystal according to claim 2, wherein the crystal has characteristic peaks at chemical shifts of 125.8 ppm, 134.8 ppm, 136.7 ppm and 150.1 ppm in its solid-state NMR spectrum ($^{13}C$).

**6.** The crystal according to claim 2, wherein its solid-state NMR spectrum ($^{13}C$) has a pattern shown in Figure 3.

**7.** The crystal according to claim 2, wherein the crystal has characteristic peaks at wave numbers of 1313 $cm^{-1}$, 1324 $cm^{-1}$, and 1391 $cm^{-1}$ in its infrared absorption spectrum (KBr method).

**8.** The crystal according to claim 2, wherein its infrared absorption spectrum (KBr method) has a pattern shown in Figure 5.

**9.** The crystal according to any one of claims 2 to 8, wherein its endothermic peak in thermogravimetry/differential thermal analysis is at 234°C.

**10.** The crystal according to claim 1, wherein the crystal has a crystal form B.

**11.** The crystal according to claim 10, wherein the crystal has characteristic peaks at diffraction angles of 2θ = 10.8°, 12.0°, 13.8°, 16.0°, 19.3°, 21.0°, 22.4°, 23.7°, 26.6°, and 31.2° in its X-ray powder diffraction spectrum.

**12.** The crystal according to claim 10, wherein its X-ray powder diffraction spectrum has a pattern shown in Figure 2.

**13.** The crystal according to claim 10, wherein the crystal has characteristic peaks at chemical shifts of 16.4 ppm (double

peak), 120.5 ppm, and 135.6 ppm in its solid-state NMR spectrum ($^{13}C$).

**14.** The crystal according to claim 10, wherein its solid-state NMR spectrum ($^{13}C$) has a pattern shown in Figure 4.

**15.** The crystal according to claim 10, wherein the crystal has characteristic peaks at wave numbers of 1329 $cm^{-1}$, 1382 $cm^{-1}$, and 1401 $cm^{-1}$ in its infrared absorption spectrum (KBr method).

**16.** The crystal according to claim 10, wherein its infrared absorption spectrum (KBr method) has a pattern shown in Figure 6.

**17.** The crystal according to any one of claims 10 to 16, wherein its endothermic peak in thermogravimetry/differential thermal analysis is at 236°C.

**18.** A method for producing a crystal of 2-(5-chloropyridin-3-yl)-1-(2,5-dichlorobenzyl)-4-methyl-1H-imidazole-5-carboxylic acid, comprising the steps of:

suspending an alkyl ester of 2-(5-chloropyridin-3-yl)-1-(2,5-dichlorobenzyl)-4-methyl-1H-imidazole-5-carboxylic acid in a solvent and performing hydrolysis by adding an aqueous solution of a base thereto; and
neutralizing the reaction product.

**19.** The method for producing a crystal according to claim 18, further comprising the step of stirring the neutralized product while cooling.

**20.** The method for producing a crystal according to claim 18 or 19, wherein the solvent is a single organic solvent selected from methanol, ethanol, 2-propanol, 1-butanol, and tetrahydrofuran, or a mixture solvent of the organic solvent and water, or a mixture of two or more organic solvents selected from methanol, ethanol, 2-propanol, and 1-butanol, or a mixture solvent of the mixture of the organic solvents and water.

**21.** The method for producing a crystal according to claim 20, wherein the solvent is a single organic solvent selected from methanol, ethanol, 2-propanol, and 1-butanol, or a mixture solvent of the single organic solvent and water.

**22.** The method for producing a crystal according to claim 21, wherein the solvent is ethanol.

**23.** The method for producing a crystal according to claim 18 or 19, wherein the solvent is a mixture solvent of tetrahydrofuran and ethanol.

**24.** A pharmaceutical composition comprising the crystal according to any one of claims 1 to 17 and a pharmaceutically acceptable carrier.

**25.** A URAT1 inhibitor comprising the crystal according to any one of claims 1 to 17 as an active ingredient.

**26.** A therapeutic or prophylactic agent for one or more diseases selected from the group consisting of gout, hyperuricemia, hypertension, renal disease, diabetes, arteriosclerosis, and Lesch-Nyhan syndrome, comprising the crystal according to any one of claims 1 to 17 as an active ingredient.

FIG. 1

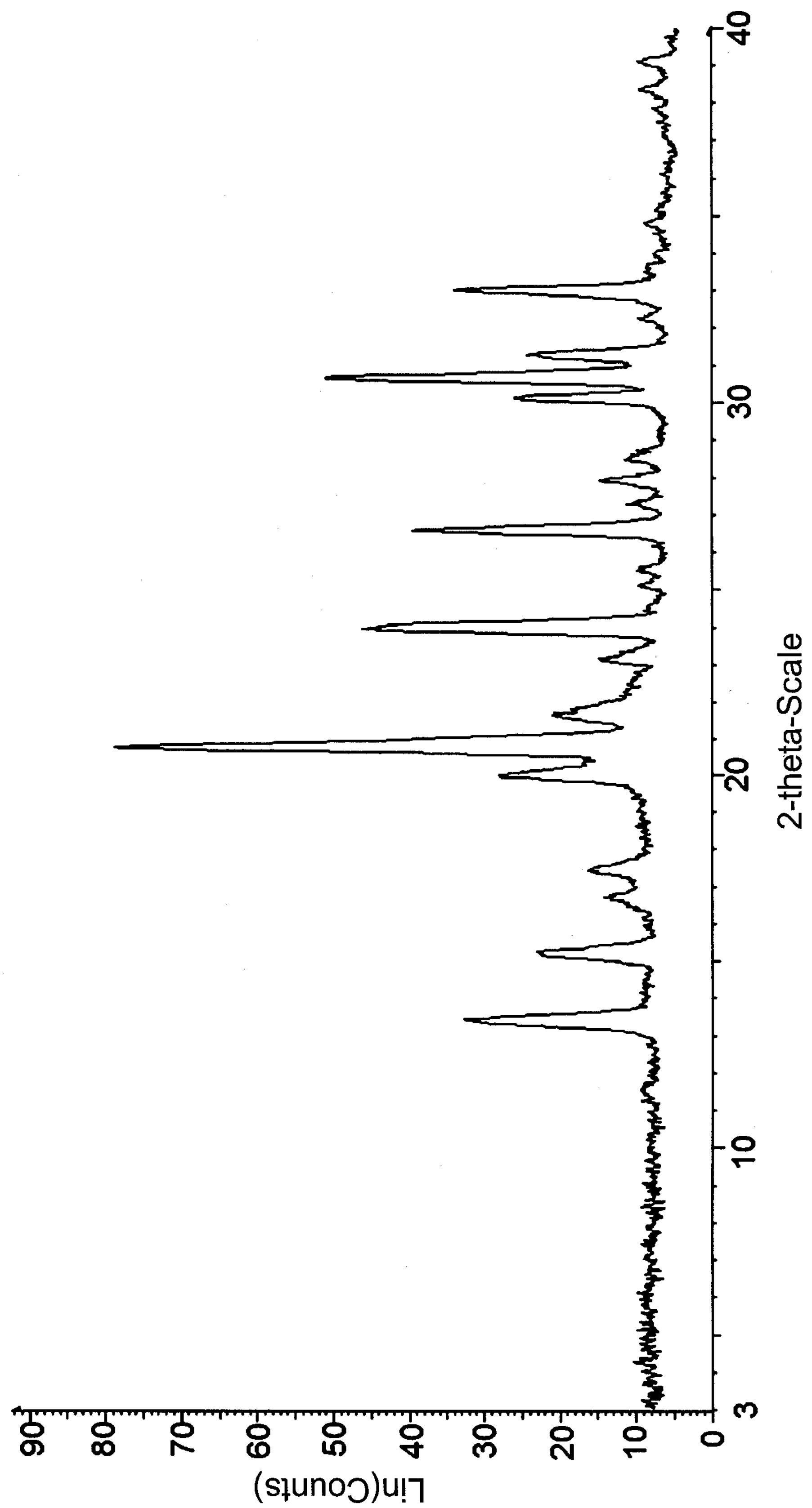

FIG. 2

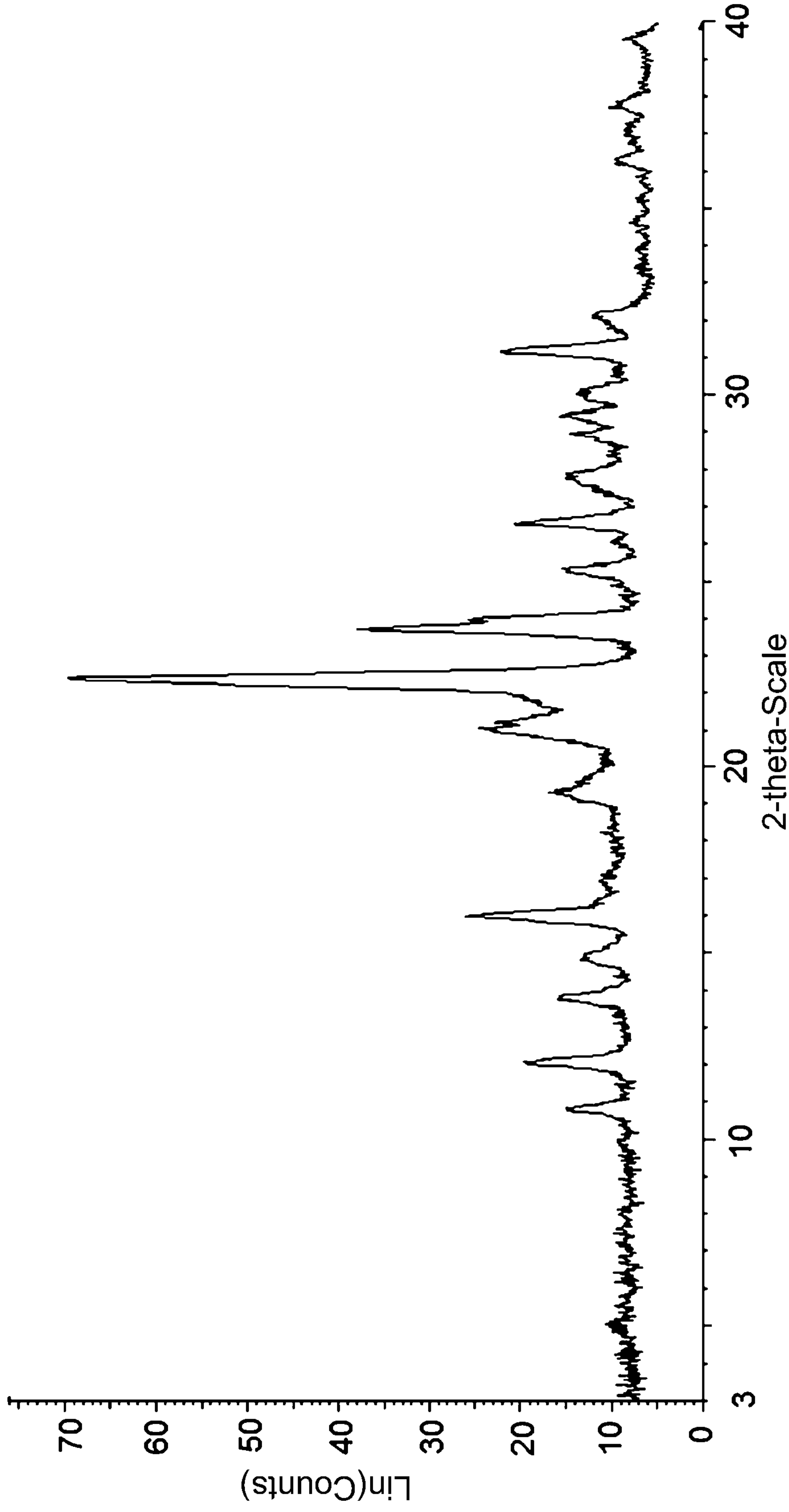

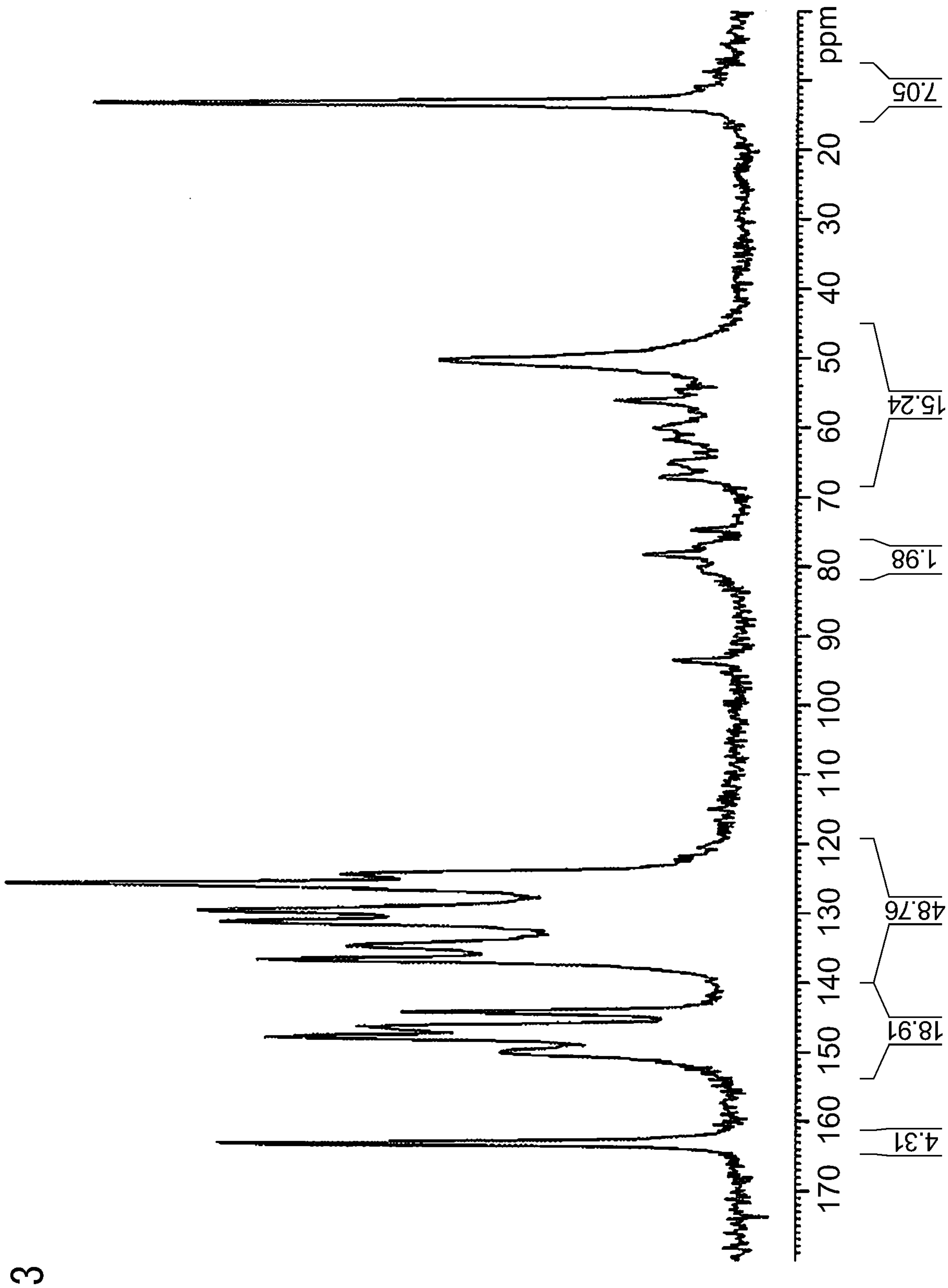
ppm
20
30
40
50
60
70
80
90
100
110
120
130
140
150
160
170
7.05
15.24
1.98
48.76
18.91
4.31

FIG. 3

FIG. 4

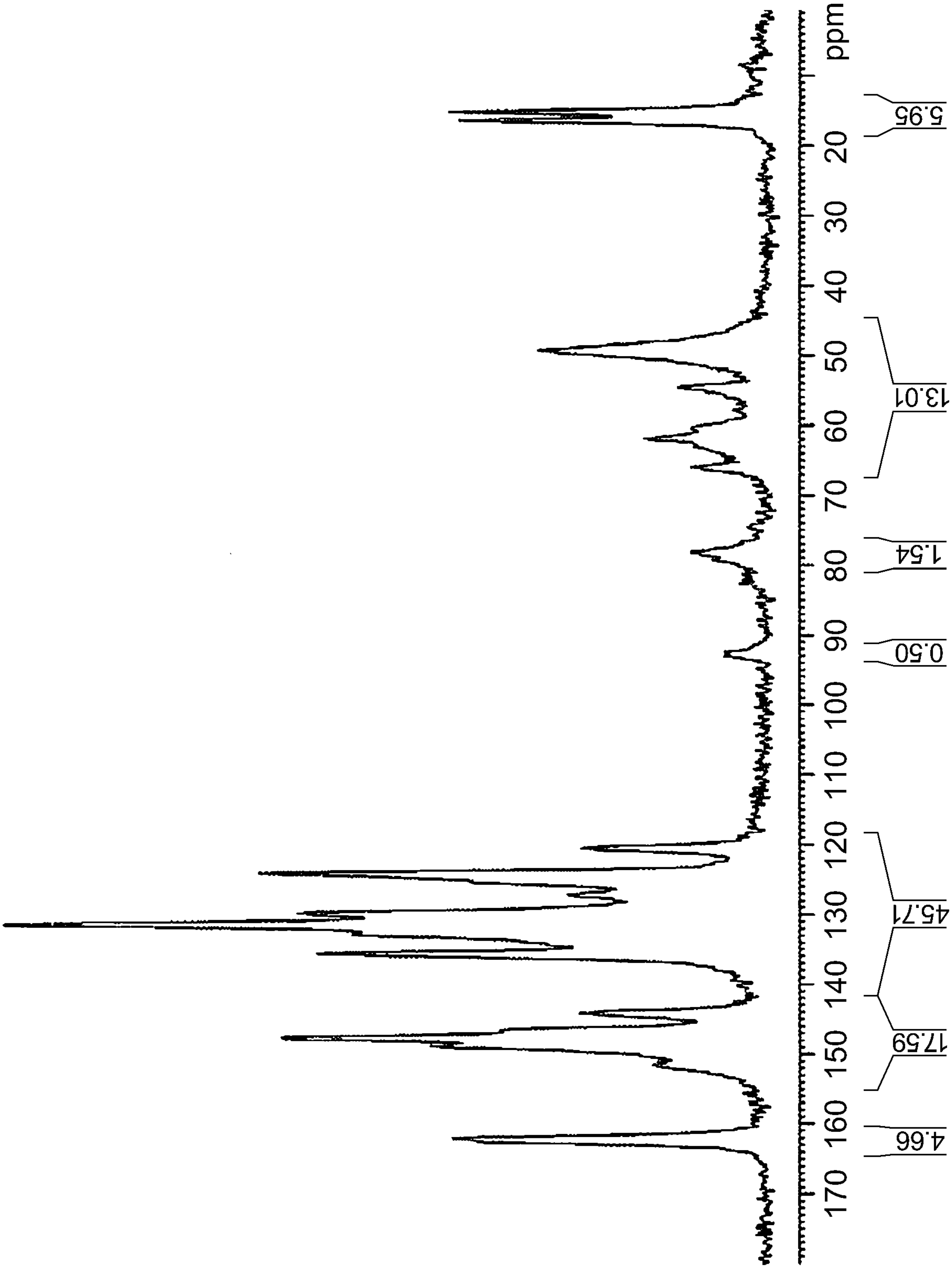

ppm
20
30
40
50
60
70
80
90
100
110
120
130
140
150
160
170
5.95
13.01
1.54
0.50
45.71
17.59
4.66

FIG. 5

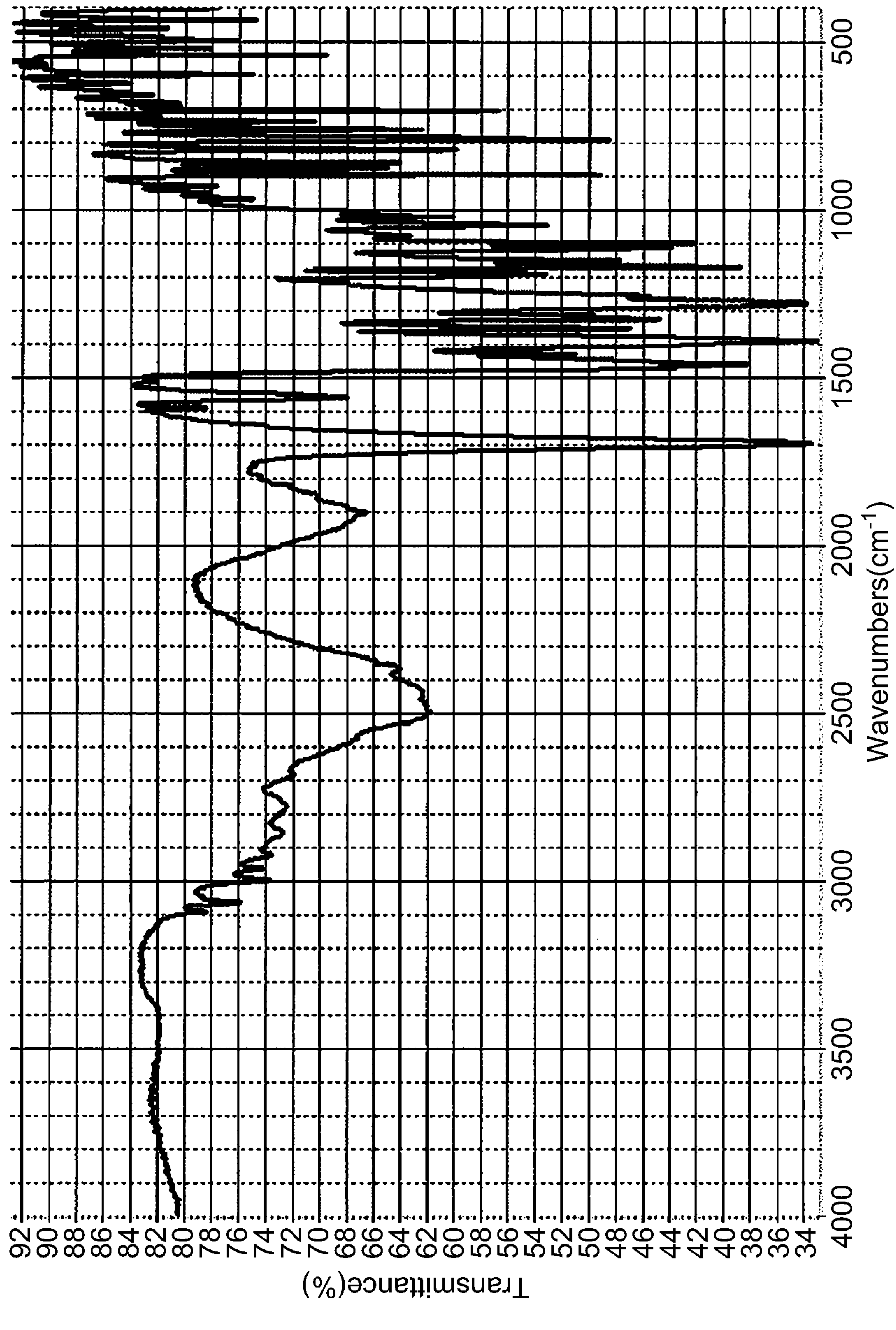
Transmittance(%)
92 90 88 86 84 82 80 78 76 74 72 70 68 66 64 62 60 58 56 54 52 50 48 46 44 42 40 38 36 34
Wavenumbers(cm-1)
4000 3500 3000 2500 2000 1500 1000 500

FIG. 6

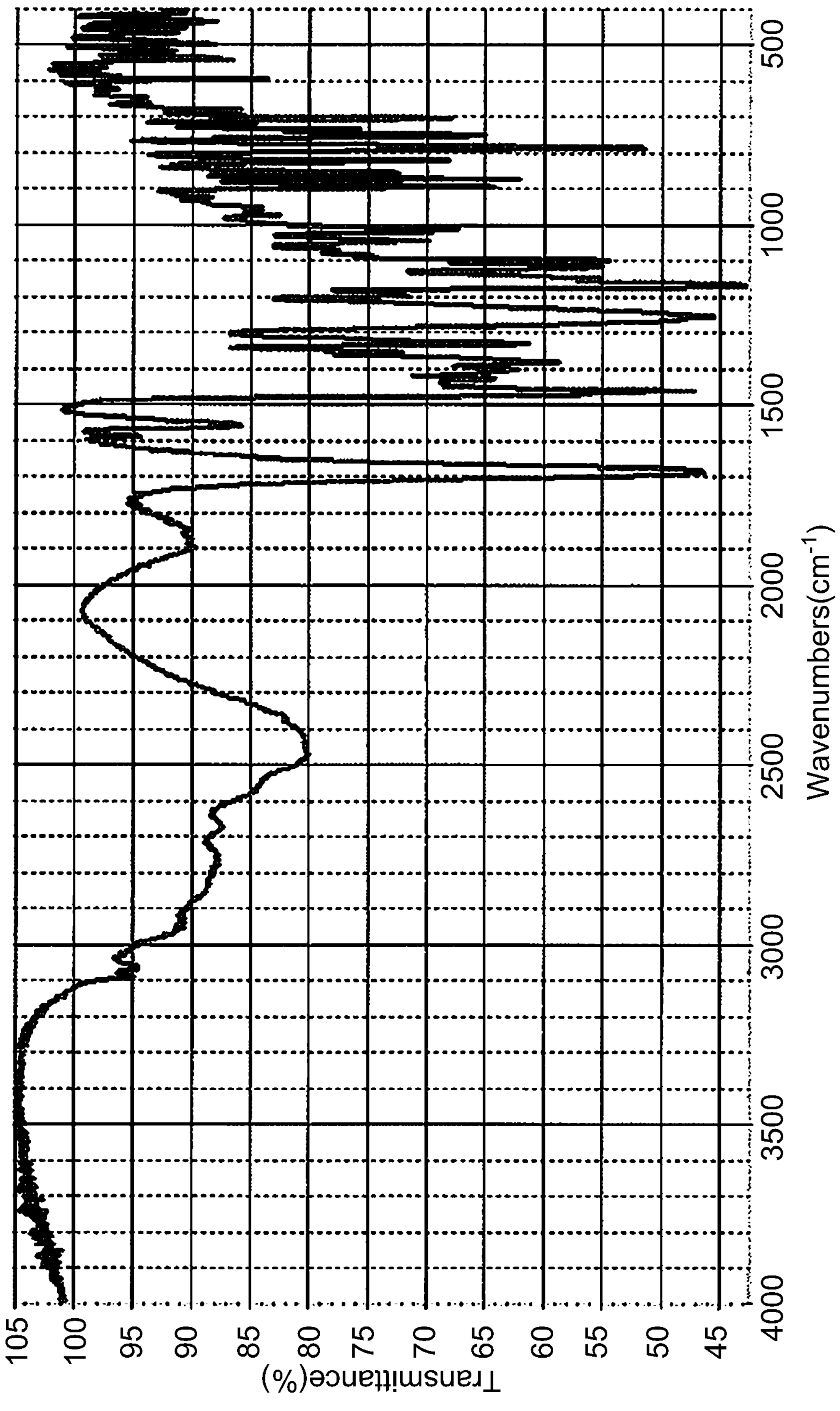
Transmittance(%)
105
100
95
90
85
80
75
70
65
60
55
50
45
Wavenumbers(cm-1)
4000
3500
3000
2500
2000
1500
1000
500

## INTERNATIONAL SEARCH REPORT

International application No.
PCT/JP2015/063632

**A. CLASSIFICATION OF SUBJECT MATTER**

*C07D401/04*(2006.01)i, *A61K31/4439*(2006.01)i, *A61P3/10*(2006.01)i, *A61P9/10*(2006.01)i, *A61P9/12*(2006.01)i, *A61P13/12*(2006.01)i, *A61P19/06*(2006.01)i, *A61P43/00*(2006.01)i

According to International Patent Classification (IPC) or to both national classification and IPC

**B. FIELDS SEARCHED**

Minimum documentation searched (classification system followed by classification symbols)
C07D401/04, A61K31/4439, A61P3/10, A61P9/10, A61P9/12, A61P13/12, A61P19/06, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| A | WO 2007/148787 A1 (Teijin Pharma Ltd.), 27 December 2007 (27.12.2007), claim 1; examples & JP 5193863 B & US 2009/0203919 A1 & EP 2039691 A1 | 1-26 |
| A | WO 2011/007895 A1 (Teijin Pharma Ltd.), 20 January 2011 (20.01.2011), claims 1 to 4 & JP 5654462 B & US 2012/0108821 A1 & EP 2455372 A1 & CN 102471295 A & KR 10-2012-0036363 A | 1-26 |
| A | JP 2011-020950 A (Mitsutaka KITAMURA), 03 February 2011 (03.02.2011), claims 1 to 2; examples (Family: none) | 1-26 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 June 2015 (01.06.15) | 16 June 2015 (16.06.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No.
PCT/JP2015/063632

C (Continuation). DOCUMENTS CONSIDERED TO BE RELEVANT

| Category* | Citation of document, with indication, where appropriate, of the relevant passages | Relevant to claim No. |
|---|---|---|
| A | WO 2010/098428 A1 (Teijin Pharma Ltd.), 02 September 2010 (02.09.2010), claims 1 to 21; examples & JP 5312570 B & US 2011/0313169 A1 & EP 2404908 A1 & EP 2754658 A1 & CN 102333765 A & KR 10-2011-0120896 A | 1-26 |
| A | WO 2012/026565 A1 (Teijin Pharma Ltd.), 01 March 2012 (01.03.2012), claims 1 to 27; examples & JP 5654601 B & US 2013/0158272 A1 & EP 2610248 A1 & CN 103068807 A & KR 10-2013-0138219 A | 1-26 |
| A | WO 2009/145456 A1 (C & C RESEARCH LABORATORIES), 03 December 2009 (03.12.2009), claims 1 to 27; examples & JP 2011-516468 A & US 2011/0028467 A1 & EP 2266989 A2 & KR 10-2011-0005688 A & CN 102015726 A | 1-26 |
| P,X | WO 2014/077285 A1 (Teijin Pharma Ltd.), 22 May 2014 (22.05.2014), claims 1 to 23; example 5 & TW 201422603 A | 1-26 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- The Guideline Revising Committee of Japanese Society of Gout and Nucleic Acid Metabolism. Medical Review, 2010
- **ENOMOTO A. et al.** *Nature,* 2002, vol. 417, 447-452